# EUROPÄISCHE PATENTSCHRIFT

(11) **EP 3 849 402 B1**
(45) Veröffentlichungstag und Bekanntmachung des Hinweises auf die Patenterteilung: **12.07.2023**
(21) Anmeldenummer: 19769750.1
(22) Anmeldetag: 12.09.2019
(51) Int. Cl.: A61B 5/00, A61B 5/01

(54) **SENSORPFLASTER MIT SCHUTZSCHICHT**
SENSOR PATCH HAVING A PROTECTIVE LAYER
PANSEMENT FORMANT CAPTEUR COMPORTANT UNE COUCHE DE PROTECTION

(30) Priorität: 13.09.2018 DE 102018122420; 22.07.2019 DE 102019119701
(43) Veröffentlichungstag der Anmeldung: 21.07.2021
(73) Patentinhaber: Steadysense GmbH, 8054 Seiersberg-Pirka (AT)
(72) Erfinder: KOELE, Werner, 8501 Lieboch (AT); GASTEINER, Peter, 8042 Graz (AT)
(74) Vertreter: Canzler & Bergmeier Patentanwälte Partnerschaft mbB
(86) Internationale Anmeldenummer: PCT/EP2019/074294
(87) Internationale Veröffentlichungsnummer: WO 2020/053317

(56) Entgegenhaltungen:
- US-A1- 2009 234 200
- US-A1- 2016 183 794
- US-A1- 2018 028 070

## Beschreibung

Die vorliegende Erfindung betrifft eine Messvorrichtung zum Messen einer Körpertemperatur eines Lebewesens, insbesondere eines Menschen, mit einer Messeinheit zum Messen der Körpertemperatur.

Aus der DE 10 2015 010 189 A1 ist eine Vorrichtung zum Bestimmen von wenigstens einer Körpertemperatur bekannt. Die Vorrichtung umfasst ein Erfassungspad, das eine Körperwärme zu einem Halbleiterchip leitet, der die Körpertemperatur ermittelt.

Aus der US 2016/0183794 A1 ist ein aktiv betriebenes Temperaturdatenlogger-Patch mit drahtloser Datenkommunikation bekannt, das eine Batterie, eine flexible Schaltung mit einem Mikroprozessor, einen Temperatursensor, einen drahtlosen Kommunikationssender und eine Antenne umfasst. Das Pflaster ist so konfiguriert, dass es sich einer gekrümmten Oberfläche des Zielobjekts anpasst. Ein Klebstoff des Pflasters ist so konfiguriert, dass er entfernbar auf die Haut des Patienten aufgetragen wird. Das Pflaster umfasst ferner eine Hydrogelschicht, die die Wärmeleitfähigkeit zwischen dem Temperatursensor und der Haut des Benutzers erhöht.

Aus der US 2009/0234200 A1 und US 2018/0028070 A1 sind weitere Temperaturdatenlogger-Patches bekannt, die ein wärmeleitendes metallisches Element aufweisen, das unmittelbar an einem Körper, an dem das Patch befestigt ist, anliegt.

Aufgabe der vorliegenden Erfindung ist es, Messvorrichtungen des Stands der Technik zu verbessern.

Die Aufgabe wird gelöst durch eine Messvorrichtung zum Messen einer Körpertemperatur eines Lebewesens mit den Merkmalen der unabhängigen Patentansprüche.

Vorgeschlagen wird eine Messvorrichtung zum Messen einer Körpertemperatur eines Lebewesens. Das Lebewesen kann beispielsweise ein Mensch sein. Die Messvorrichtung kann beispielsweise für medizinische Zwecke verwendet werden. Beispielsweise kann mit Hilfe der Messvorrichtung eine Ovulation ermittelt werden. Die Messvorrichtung kann aber auch für gesundheitlich kranke Lebewesen und/oder Personen verwendet werden, um ihren Gesundheitszustand überwachen zu können. Die Messvorrichtung kann beispielsweise auf der Haut des Lebewesens angeordnet sein, um die Körpertemperatur zu messen. Die Messvorrichtung kann beispielsweise an einem Arm oder dem Rumpf der Person angeordnet werden. Zusätzlich oder alternativ kann die Messvorrichtung auch über einer Wunde angeordnet sein, um beispielsweise die Wunde vor Schmutz zu schützen und um den Heilprozess zu unterstützen.

Die Messvorrichtung umfasst eine Messeinheit zum Messen der Körpertemperatur. Die Messeinheit kann beispielsweise eine Sensoreinheit umfassen, mit der die Körpertemperatur ermittelt werden kann. Die Messeinheit und/oder die Sensoreinheit kann beispielsweise einen Temperaturfühler und/oder einen Temperatursensor umfassen.

Erfindungsgemäß weist die Messvorrichtung eine erste Klebeschicht zum Befestigen der Messvorrichtung am Körper des Lebewesens auf. Die erste Klebeschicht kann dabei vorteilhafterweise ebenfalls hautverträglich sein. Dabei kann die erste Klebeschicht an einer dem dafür vorgesehenen Körper zugewandte Seite der Messvorrichtung angeordnet sein. Ebenso kann die erste Klebeschicht an der dem Körper zugewandten Unterseite der Messvorrichtung angeordnet sein. Mit Hilfe der ersten Klebeschicht kann die Messvorrichtung am Körper für eine längere Zeit befestigt werden. Die Unterseite kann die dem Körper zugewandte Seite der Messvorrichtung sein.

Des Weiteren weist die erfindungsgemäße Messvorrichtung eine hautverträgliche Schutzschicht auf. Die hautverträgliche Schutzschicht ist dabei zwischen der ersten Klebeschicht und der Messeinheit angeordnet. Insbesondere ist die Schutzschicht somit zwischen der Haut des Lebewesens und der Messeinheit angeordnet. Da die Messeinheit beispielsweise einen Halbleiterchip umfassen kann, kann dieser Materialien aufweisen, welche gesundheitsgefährdend oder hautreizend sein können. Insbesondere kann der Halbleiterchip nicht darauf geprüft sein, ob dieser aufgrund der enthaltenen Materialien für medizinische Zwecke verwendet werden darf. Mit Hilfe der hautverträglichen Schutzschicht kommt die Messeinheit nicht in Kontakt mit dem Körper bzw. mit der Haut des Lebewesens, so dass beispielsweise auch die ungeprüften Halbleiterchips verwendet werden können. Durch die hautverträgliche Schutzschicht ist die Messeinheit vor der Haut abgekapselt. Durch die hautverträgliche Schutzschicht kann eine Reizung, beispielsweise durch eine allergische Reaktion, der Haut des Körpers des Lebewesens durch die Messeinheit vermieden werden.

Die Schutzschicht weist dabei eine Dicke auf, so dass ein Wärmefluss zwischen dem Körper und der Messeinheit nur wenig beeinflusst ist. Die Dicke der Schutzschicht kann beispielsweise im Bereich zwischen 0,001 mm und 1 mm liegen. Beispielsweise weist die Schutzschicht eine Dicke von 25 µm, 50 µm oder 75 µm auf.

Beispielsweise kann die Messeinheit und/oder die Sensoreinheit eine temperaturabhängige Diode und/oder einen temperaturabhängigen Widerstand umfassen, mittels der die Körpertemperatur ermittelbar ist.

Die Messeinheit kann ferner beispielsweise von einem Lack abgekapselt sein, der eine hohe Flüssigkeits- und/oder Gasdiffusionsbarriere ausbildet, so dass beispielsweise eine Korrosion der Messeinheit verhindert bzw. vermindert werden kann. Der Lack kann auch wasser- und/oder gasdicht sein.

Die Messvorrichtung weist ein Wärmeleitelement auf, mittels dem beim bestimmungsgemäßen Gebrauch der Messvorrichtung ein Wärmefluss zwischen einem Körper des Lebewesens und der Messeinheit ausbildbar ist. Das Wärmeleitelement weist eine Wärmeleitfähigkeit auf. Mit Hilfe des Wärmeleitelements bzw. durch den Wärmefluss, der durch das Wärmeleitelement ausbildbar ist, kann ein Temperaturunterschied zwischen dem Körper und der Messeinheit ausgeglichen werden. Dabei wird angenommen, dass der Körper des Lebewesens sehr viel größer ist, d.h. eine sehr viel größere Masse aufweist, als die Messvorrichtung, so dass die Messeinheit stets die Temperatur des Körpers annimmt. Die Temperatur des Körpers des Lebewesens bleibt somit durch den Kontakt mit der Messvorrichtung im Wesentlichen unbeeinflusst. Hat die Messeinheit die Temperatur des Körpers angenommen, kann die Körpertemperatur gemessen werden. Dabei kann das Wärmeleitelement den Wärmefluss zwischen dem Körper und der Messeinheit in beide Richtungen ausbilden. Das heißt, wenn der Körper wärmer ist als die Messeinheit, leitet das Wärmeleitelement Wärme vom Körper zur Messeinheit. Dies erfolgt solange, bis der Temperaturunterschied zwischen Körper und Messeinheit ausgeglichen ist. Ist dagegen die Messeinheit wärmer als der Körper, leitet das Wärmeleitelement Wärme von der Messeinheit zum Körper bis der Temperaturunterschied ausgeglichen ist. Erfolgt keine Änderung der Temperatur der Messeinheit, weisen die Messeinheit und der Körper dieselbe Temperatur auf, so dass die Körpertemperatur ermittelt werden kann.

Das Wärmeleitelement ist zwischen der hautverträglichen Schutzschicht und der Messeinheit angeordnet. Dadurch kann das Wärmeleitelement die Wärme zwischen dem Körper und der Messeinheit austauschen. Dabei ist die hautverträgliche Schutzschicht beim verwendungsgemäßen Gebrauch der Messvorrichtung zwischen dem Körper und dem Wärmeleitelement angeordnet. Infolgedessen kann beispielsweise eine Hautreizung des Körpers durch das Wärmeleitelement verhindert werden. Auch können ungeprüfte Materialien des Wärmeleitelements verwendet werden.

Von Vorteil ist es, wenn zwischen der hautverträglichen Schutzschicht und der Messeinheit eine zweite Klebeschicht angeordnet ist. Zusätzlich oder alternativ kann auch zwischen der hautverträglichen Schutzschicht und dem Wärmeleitelement die zweite Klebeschicht angeordnet sein. Dadurch haftet die Schutzschicht an der Messeinheit bzw. am Wärmeleitelement.

Wenn zwischen der Schutzschicht und der Messeinheit und/oder dem Wärmeleitelement die zweite Klebeschicht angeordnet ist, ist es vorteilhaft, wenn die Schutzschicht als doppelseitig klebende hautverträgliche Schutzschicht ausgebildet ist. Die Schutzschicht weist dann auf der einen Seite die erste Klebeschicht und auf der dazu gegenüberliegenden Seite die zweite Klebeschicht auf. Die erste Klebeschicht, die hautverträgliche Schutzschicht und die zweite Klebeschicht können einteilig als, insbesondere doppelseitig klebender, Klebe-/Schutzschicht-Verbund ausgebildet sein.

Die Messvorrichtung weist eine Außenschicht auf, die an einer beim verwendungsgemäßen Gebrauch dem Körper gegenüberliegenden Oberseite der Messvorrichtung angeordnet ist. Die Außenschicht kann beispielsweise als eine Textilschicht ausgebildet sein, so dass ein Tragekomfort der Messvorrichtung verbessert ist.

Weiterhin ist es vorteilhaft, wenn die Messvorrichtung eine Trägereinheit aufweist, in der die Messeinheit angeordnet ist. Zusätzlich oder alternativ kann die Messeinheit auch auf der Trägereinheit angeordnet sein. Zusätzlich oder alternativ kann in der Trägereinheit auch das Wärmeleitelement angeordnet sein. Zusätzlich oder alternativ kann das Wärmeleitelement auch auf der Trägereinheit angeordnet sein. Die Trägereinheit kann somit die Messeinheit und/oder das Wärmeleitelement aufnehmen, um diese, insbesondere in der Messvorrichtung, zu fixieren. Die Trägereinheit kann beispielsweise scheibenförmig ausgebildet sein, so dass die Messvorrichtung im Wesentlichen scheibenförmig ausgebildet ist. Die Trägereinheit kann ferner aus einem flexiblen und/oder elastischen Material ausgebildet sein, so dass sich die Trägereinheit bzw. die Messvorrichtung an unterschiedliche Körperkonturen des zu messenden Körpers anpassen kann. Die Trägereinheit kann beispielsweise aus einem flexiblen Polymermaterial, beispielsweise auf Basis von Kohlenstoff oder Silizium, hergestellt sein. Das Polymermaterial kann beispielsweise ein Kunststoff und/oder ein Silikon sein. Beispielsweise kann die Trägereinheit auch eine Leiterplatte sein, an der die Messeinheit angeordnet ist. Die Leiterplatte kann elektrische Leitungen aufweisen. Die Leiterplatte kann ebenfalls flexibel sein. Über die Trägereinheit kann die Messeinheit beispielsweise mit einer elektrischen Spannung versorgt werden. Die Trägereinheit kann ebenfalls eine Antenne aufweisen, um die gemessene Körpertemperatur drahtlos übermitteln zu können. Ist die Trägereinheit die Leiterplatte kann ferner die Messeinheit beispielsweise auf dieser aufgelötet sein. Mit Hilfe der Leiterplatte kann die Messeinheit auch mit der Antenne verbunden sein.

Ferner ist es von Vorteil, wenn das Wärmeleitelement ein Flächenelement umfasst, das auf einer beim bestimmungsgemäßen Gebrauch der Messvorrichtung dem Körper zugewandten Seite der Messeinheit angeordnet ist. Das Flächenelement ist flächig ausgebildet, so dass das Flächenelement in einem entsprechend großen Bereich Wärmekontakt mit dem Körper bzw. mit der Haut des Lebewesens aufweist. Dadurch kann der Wärmefluss des Wärmeleitelements verbessert werden, so dass beispielsweise in kurzer Zeit der Temperaturunterschied zwischen der Messeinheit und dem Körper ausgeglichen werden kann. Infolgedessen kann die Körpertemperatur entsprechend schnell gemessen werden. Das Flächenelement kann dabei unmittelbaren Kontakt mit der Messeinheit aufweisen. Das Flächenelement kann somit unmittelbar mit der Messeinheit verbunden sein. Das Flächenelement kann somit eine Wärmeleitschicht auf der Messeinheit ausbilden. Das Flächenelement ist somit an der Messeinheit angeordnet. Die Messeinheit und das Flächenelement können auch einteilig miteinander ausgebildet sein. Das Flächenelement kann beispielsweise zumindest teilweise in der Messeinheit angeordnet sein.

Vorteilhaft ist es des Weiteren, wenn das Wärmeleitelement eine Durchkontaktierung umfasst. Die Durchkontaktierung kann sich beispielsweise von der Messeinheit zur im bestimmungsgemäßen Gebrauch dem Körper zugewandten Seite der Messvorrichtung erstrecken. Die Durchkontaktierung kann sich beispielsweise bis zur Schutzschicht erstrecken. Die Durchkontaktierung kann sich dabei beispielsweise durch die Trägereinheit hindurch erstrecken, so dass der Wärmefluss zwischen dem Körper und der Messeinheit durch die Trägereinheit ausgebildet werden kann. Die Trägereinheit kann beispielsweise einen Kontaktierungsdurchbruch aufweisen, durch den sich die Durchkontaktierung, insbesondere von der Messeinheit in Richtung des Körpers, hindurcherstreckt. Mit Hilfe der Durchkontaktierung kann ebenfalls der Wärmefluss zwischen dem Körper des Lebewesens und der Messeinheit ausgebildet werden. Die Durchkontaktierung kann außerdem zwischen dem Flächenelement und der Messeinheit angeordnet sein, so dass das Flächenelement von der Messeinheit beabstandet ist. Dadurch umfasst das Wärmeleitelement zumindest das Flächenelement und die Durchkontaktierung. Mit Hilfe der Durchkontaktierung kann beispielsweise die Messeinheit von der Unterseite der Messvorrichtung beabstandet angeordnet sein. Die Durchkontaktierung kann somit den Wärmefluss zwischen dem Flächenelement und der Messeinheit ausbilden.

Wenn das Flächenelement einen größeren Querschnitt als die Durchkontaktierung aufweist, bringt dies ebenfalls Vorteile mit sich. Dadurch weist die Durchkontaktierung eine Flexibilität auf, insbesondere kann sich die Durchkontaktierung verbiegen, so dass sich das Wärmeleitelement, welches das Flächenelement und die Durchkontaktierung aufweist, an die Körperkontur anpassen kann. Da die Durchkontaktierung aufgrund des im Vergleich zum Flächenelement geringeren Querschnitts flexibel ist, kann sich das Flächenelement verschwenken, um sich an der Körperkontur anzupassen. Beispielsweise sollte, um einen hohen Wärmefluss zwischen Körper und Messeinheit ausbilden zu können, das Flächenelement im Wesentlichen parallel zur Körperoberfläche bzw. zur Haut orientiert sein.

Außerdem kann mit dem Flächenelement der Wärmefluss erhöht werden, da dieser auch von der Querschnittsfläche des Flächenelements abhängt.

Zusätzlich oder alternativ kann die Flexibilität der Durchkontaktierung auch dadurch ausgebildet werden, dass diese eine bestimmte Form aufweist. Beispielsweise kann die Durchkontaktierung eine Einschnürung aufweisen, so dass die Flexibilität ausgebildet ist.

Zusätzlich oder alternativ kann die Flexibilität der Durchkontaktierung auch durch die Materialwahl der Durchkontaktierung erreicht werden. Beispielsweise kann die Durchkontaktierung aus einem flexiblen Material ausgebildet sein. Selbstverständlich sollte die Wahl des Materials die Wärmeleitfähigkeit der Durchkontaktierung nur wenig oder derart beeinflussen, dass die Funktionsfähigkeit der Messvorrichtung wenig oder nicht beeinträchtigt wird.

Ferner kann die Durchkontaktierung aus einem zum Flächenelement unterschiedlichen Material ausgebildet sein. Beispielsweise kann die Durchkontaktierung aus einem Material hergestellt sein, dass im Vergleich zum Material des Flächenelements eine höhere Wärmeleitfähigkeit aufweist. Dadurch kann beispielsweise der geringere Querschnitt der Durchkontaktierung im Vergleich zum Flächenelement ausgeglichen werden. Dabei braucht bei der Wahl des Materials der Durchkontaktierung weniger Rücksicht auf eine Hautverträglichkeit genommen werden, da sich die Durchkontaktierung in einem inneren Bereich der Messvorrichtung befinden kann. Infolgedessen stehen mehr unterschiedliche Materialien zur Verfügung, die beispielsweise die im Vergleich zum Flächenelement höhere Wärmeleitfähigkeit aufweisen und/oder kostengünstiger sind.

Von Vorteil ist es auch, wenn sich die Schutzschicht vollständig über die Unterseite der Messvorrichtung erstreckt. Dadurch ist beispielsweise eine Reizung durch die Messvorrichtung vermindert. Die Unterseite ist dabei im bestimmungsgemäßen Gebrauch der Messvorrichtung dem Körper zugewandt. Zusätzlich oder alternativ kann sich auch die Klebeschicht vollständig über die Unterseite der Messvorrichtung erstrecken. Dadurch haftet die Messvorrichtung sicher auf dem Körper.

Vorteilhaft ist es, wenn die Schutzschicht zumindest im Bereich des Wärmeleitelements zumindest ein Wärmekontaktelement und/oder Zusatzstoffe aufweist, mittels denen der Wärmefluss erhöht werden kann. Zusätzlich oder alternativ ist es vorteilhaft, wenn die Klebeschicht zumindest im Bereich des Wärmeleitelements zumindest ein Wärmekontaktelement und/oder Zusatzstoffe aufweist, mittels denen der Wärmefluss erhöht werden kann. Das Wärmekontaktelement und/oder die Zusatzstoffe erhöhen somit die Wärmeleitfähigkeit, so dass ein Temperaturunterschied zwischen dem Körper des Lebewesens und der Messeinheit schneller ausgleichbar ist. Das Wärmekontaktelement kann beispielsweise Partikel aus einem Material, beispielsweise Metalle wie Silber, Kupfer usw., mit einer hohen Wärmeleitfähigkeit umfassen. Derartige Wärmekontaktelemente können dem Material der Schutzschicht beigemischt sein. Zusätzlich oder alternativ können auch die Zusatzstoffe Metalle umfassen.

Vorteilhaft ist es, wenn die Messvorrichtung eine Versiegelung aufweist. Die Versiegelung kann dabei zumindest die Messeinheit, das Wärmeleitelement und/oder die Trägereinheit umschließen. Die Versiegelung kann beispielsweise auch eine Vergießung sein. Die Versiegelung kann auch aus einem flexiblen Material ausgebildet sein, so dass sich die Versiegelung der Körperkontur anpassen kann.

Zusätzlich oder alternativ kann die Versiegelung beispielsweise aus zumindest einer Schaumfolie ausgebildet sein, welche zumindest eine Aussparung aufweisen kann. In der Aussparung kann beispielsweise die Messeinheit angeordnet sein. Die Versiegelung kann auch aus mehreren, beispielsweise zwei, Schaumfolien zusammengesetzt sein, welche übereinander angeordnet werden und zumindest die Messeinheit abkapseln. Ein Vorteil der Schaumfolie ist, dass diese gut wärmeisolierend sind, so dass ein Wärmefluss von der Umgebung zur Messeinheit verringert ist. Die Messung wird dadurch weniger durch die Umgebung verfälscht.

Das Wärmeleitelement ist aus Metall ausgebildet. Da Metalle zumeist eine gute Wärmeleitfähigkeit aufweisen, kann dadurch auf einfache Weise das Wärmeleitelement ausgebildet werden. Außerdem ist ein Vorteil von Metall, dass das Wärmeleitelement einfacher hergestellt werden kann. Das Wärmeleitelement kann beispielsweise ausgefräst oder gegossen werden.

Zusätzlich oder alternativ kann auch die Durchkontaktierung und/oder das Flächenelement als Metall ausgebildet sein.

Des Weiteren ist es vorteilhaft, wenn die Messvorrichtung eine Energieeinheit aufweist. Die Energieeinheit kann beispielsweise eine Batterie und/oder Akkumulator aufweisen, welche die Messvorrichtung mit elektrischer Energie versorgen kann. Zusätzlich oder alternativ kann die Energieeinheit auch einen Superkondensator aufweisen, der aufgeladen ist und elektrische Energie über eine längere Zeit speichern kann.

Zusätzlich oder alternativ kann die Messvorrichtung eine Speichereinheit umfassen, mittels der Temperaturmesswerte der Messeinheit gespeichert werden können. Dadurch kann beispielsweise jede Stunde die Körpertemperatur gemessen werden, um einen Temperaturverlauf der Körpertemperatur zu erstellen. Dadurch kann ein Anstieg und/oder ein Abstieg der Körpertemperatur erkannt werden. Die Messwerte können somit zwischengespeichert werden, bis diese ausgelesen werden.

Zusätzlich oder alternativ kann die Messvorrichtung eine Schnittstelle aufweisen. Über die Schnittstelle kann beispielsweise die Körpertemperatur ausgelesen werden. An die Schnittstelle kann sich beispielsweise ein externes Auslesegerät ankoppeln, um die Körpertemperatur auszulesen. Die Schnittstelle kann dazu beispielsweile als RFID-Schnittstelle ausgebildet sein. Die Schnittstelle kann aber auch eine Bluetooth, eine WLAN und/oder eine kabelgebundene oder eine andere Schnittstelle sein. An die Schnittstelle kann beispielsweise ein Smartphone ankoppeln, um die Körpertemperatur auszulesen. Die Schnittstelle kann auch eine NFC (near field communication)-Schnittstelle sein.

Zusätzlich oder alternativ kann die Messvorrichtung und/oder die Energieeinheit auch eine Energieumwandeleinheit aufweisen. Die Energieumwandeleinheit kann beispielsweise eine oder mehrere Piezoelemente aufweisen, die eine Bewegung der Messvorrichtung in elektrische Energie umwandeln können. Dadurch kann die Messvorrichtung mit Hilfe der Bewegungsenergie betrieben werden. Zusätzlich oder alternativ kann die Energieumwandeleinheit auch ein Peltierelement umfassen, welches aus einer Temperaturdifferenz elektrische Energie erzeugt, mit der die Messvorrichtung betrieben werden kann. Zusätzlich oder alternativ kann die Energieumwandeleinheit auch eine induktive und/oder kapazitive Einheit umfassen, welche ein vorhandenes elektrisches und/oder magnetisches Feld in elektrische Energie umwandelt, so dass die Messvorrichtung betrieben werden kann.

Vorteilhaft ist es auch, wenn die Schnittstelle derart ausgebildet ist, dass die Energie zum Messen der Körpertemperatur und/oder die Energie zum Auslesen der Körpertemperatur, beispielsweise aus einem Speicher der Messeinheit, über die Schnittstelle bereitstellbar ist. Die Schnittstelle kann dazu derart ausgebildet sein, dass diese aus einem elektromagnetischen Feld, welches beispielsweise das externe Auslesegerät ausbildet, die elektrische Energie entnimmt und dadurch die Körpertemperatur ermittelt und/oder ausgelesen werden kann. Das Auslesen der Körpertemperatur kann dabei auch wieder vom externen Auslesegerät erfolgen. Dies hat den Vorteil, dass auf den Energiespeicher verzichtet werden kann und die Messvorrichtung kostengünstiger hergestellt werden kann und in der Lebenszeit im Wesentlichen unbegrenzt ist. Die Schnittstelle kann beispielsweise die NFC-Schnittstelle sein.

Von Vorteil ist es auch, wenn die Versiegelung und die Schutzschicht aus demselben Material sind. Dadurch kann beispielsweise die Schutzschicht und die Versiegelung einteilig ausgebildet werden.

Vorteilhaft ist es daneben, wenn die Versiegelung aus einem Schaum und/oder aus einem Silikon ausgebildet ist. Der Schaum kann beispielsweise eine Polyethylenschaum sein. Dadurch kann die Versiegelung wärmeisolierend ausgebildet werden, um einen Wärmefluss von der Umgebung zur Messeinheit zu vermindern. Beispielsweise kann die Versiegelung aus Polyethylen bzw. einem Polyethylenschaum ausgebildet sein. Die Schutzschicht kann beispielsweise aus Polyester ausgebildet sein.

Die Versiegelung kann auch aus einer Schaumfolie, insbesondere Polyethylenschaumfolie, ausgebildet sein. In der Schaumfolie kann die Messeinheit eingekapselt sein.

Vorteilhaft ist es, wenn die Versiegelung aus einem Material ausgebildet ist, welches eine geringere Wärmeleitfähigkeit aufweist als ein Material der Schutzschicht. Die Versiegelung kann dazu beispielsweise aus dem Schaum ausgebildet sein. Die Körpertemperatur wird dadurch schneller an die Messeinheit geleitet. Die Messeinheit nimmt dadurch schnell die Körpertemperatur an. Infolgedessen kann die Körpertemperatur schneller gemessen werden.

Dagegen wird eine Umgebungstemperatur weniger gut an die Messeinheit geleitet, so dass die Messeinheit überwiegend die Körpertemperatur misst.

Zusätzlich oder alternativ ist es vorteilhaft, wenn die Schutzschicht aus Polyester ausgebildet ist. Zusätzlich oder alternativ kann die Schutzschicht auch aus Polyethylen ausgebildet sein.

Ferner kann die Versiegelung aus einem Silikon und die Schutzschicht aus Polyethylen oder Polyester ausgebildet sein. Da die Schutzschicht aus Polyethylen oder Polyester ausgebildet ist, was eine im Vergleich zum Silikon gute Wärmeleitfähigkeit aufweist, kann die Messeinheit die Körpertemperatur schnell messen.

Da ferner die Versiegelung, welche die Messeinheit einkapseln kann, im Vergleich zur Schutzschicht eine schlechtere Wärmeleitfähigkeit aufweist, kann die Messeinheit von der Umgebungstemperatur isoliert werden. Die Messeinheit misst dadurch die Körpertemperatur und nicht die Umgebungstemperatur. Infolgedessen wird die Messgenauigkeit der Körpertemperatur verbessert, da Einflüsse der Umgebungstemperatur vermindert sind.

Weitere Vorteile der Erfindung sind in den nachfolgenden Ausführungsbeispielen beschrieben. Es zeigen:
- **Figur 1**: eine schematische Schnittansicht einer Messvorrichtung zum Messen einer Körpertemperatur mit einer Messeinheit, einer Klebeschicht und einer Schutzschicht,
- **Figur 2**: eine schematische Schnittansicht einer Messvorrichtung zum Messen einer Körpertemperatur mit einer Messeinheit und einem Wärmeleitelement,
- **Figur 3**: eine schematische Schnittansicht einer Messvorrichtung zum Messen einer Körpertemperatur mit einer Trägereinheit,
- **Figur 4**: eine schematische Schnittansicht einer Messvorrichtung zum Messen einer Körpertemperatur mit einer Trägereinheit und einem Wärmeleitelement,
- **Figur 5**: eine schematische Schnittansicht einer Messvorrichtung zum Messen einer Körpertemperatur mit einer Energieeinheit und einer Schnittstelle und
- **Figur 6**: eine schematische Schnittansicht einer Messvorrichtung zum Messen einer Körpertemperatur mit einer Energieeinheit und einer Schnittstelle.

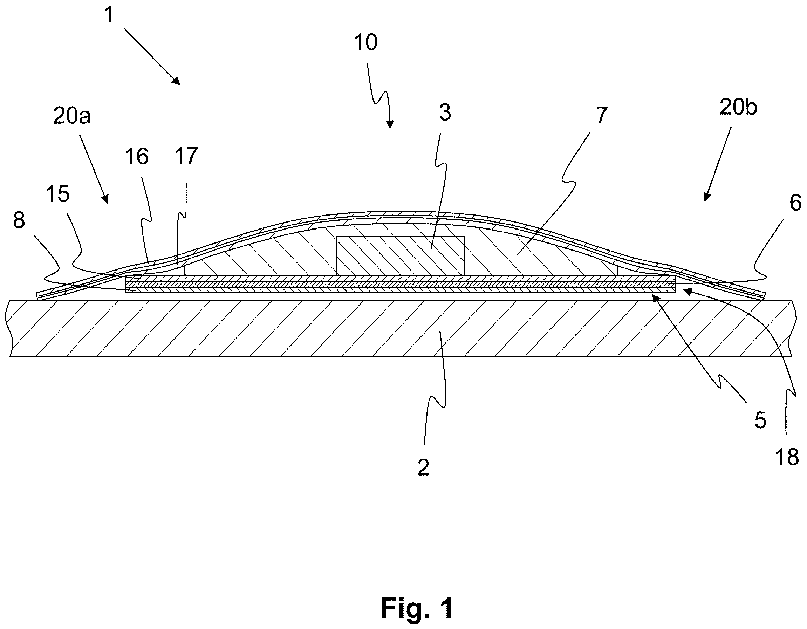

Figur 1 zeigt eine schematische Ansicht einer Messvorrichtung 1 zum Messen einer Körpertemperatur eines Körpers 2 eines Lebewesens mit einer Messeinheit 3, einer ersten hautverträglichen Klebeschicht 8 und einer hautverträglichen Schutzschicht 6. Dabei ist die Messvorrichtung 1 der Übersichtlichkeit wegen vom Körper 2 beabstandet dargestellt. Wenn die Messvorrichtung 1 bestimmungsgemäß verwendet wird, weist diese vorteilhafterweise einen Kontakt zum Körper 2 auf.

Die Figuren sind der Übersichtlichkeit wegen nicht maßstabsgetreu dargestellt. Beispielsweise sind die genannten Klebeschichten, die Schutzschicht usw. deutlich dicker dargestellt.

Mit Hilfe der Messeinheit 3 kann die Körpertemperatur des Körpers 2 gemessen bzw. ermittelt werden. Dabei kann die Körpertemperatur dadurch gemessen werden, dass die Messeinheit 3 beim Kontakt mit dem Körper 2 selbst die Körpertemperatur annimmt. Die Messeinheit 3 kann zum Messen der Temperatur beispielsweise ein hier nicht gezeigtes Sensorelement und/oder einen Temperaturfühler aufweisen. Beispielsweise kann die Messeinheit 3 eine temperaturabhängige Diode und/oder einen temperaturabhängigen Widerstand aufweisen, mittels der die Körpertemperatur ermittelbar ist. Die Messeinheit 3 kann einen Halbleitersensor zur Ermittlung der Körpertemperatur umfassen. Zusätzlich kann die Messeinheit 3 auch eine hier nicht gezeigte Recheneinheit aufweisen, mittels der die von der Messeinheit 3, vom Halbleitersensor, von der temperaturabhängigen Diode, vom Sensorelement und/oder vom Temperaturfühler erfassten Werte in einen Temperaturwert umgesetzt werden können. Beispielsweise kann das Sensorelement einen temperaturabhängigen Widerstand umfassen, wobei die Recheneinheit aus der Veränderung eines Stromflusses durch das Sensorelement die Temperatur ermittelt.

Die Körpertemperatur des Körpers 2 kann beispielsweise dadurch gemessen werden, dass die Messeinheit 3 die gleiche Temperatur wie der Körper 2 aufweist.

Die Messvorrichtung 1 weist ferner eine Unterseite 5 auf, die beim bestimmungsgemäßen Gebrauch der Messvorrichtung 1 dem Körper 2 zugewandt ist bzw. mit diesem Kontakt aufweist. Außerdem weist die Messvorrichtung 1 eine Oberseite 10 auf, die beim bestimmungsgemäßen Gebrauch der Messvorrichtung 1 vom Körper 2 abgewandt ist.

Ferner weist die Messvorrichtung 1 die hautverträgliche Schutzschicht 6 auf. Die hautverträgliche Schutzschicht 6 ist an einer dem dafür vorgesehenen Körper 2 zugewandten Seite der Messvorrichtung 1 angeordnet. Die Schutzschicht 6 kann auf der Unterseite 5 bzw. im Bereich der Unterseite 5 der Messvorrichtung 1 angeordnet sein. Wenn die Messvorrichtung 1 am Körper 2 angeordnet ist, um die Körpertemperatur zu messen, ist die hautverträgliche Schutzschicht 6 zwischen der Messeinheit 3 und dem Körper 2 bzw. einer hier nicht gezeigten Haut des Körpers 2 angeordnet. Die Messeinheit 3 kann dadurch beim bestimmungsgemäßen Gebrauch der Messvorrichtung 1 vom Körper 2 beabstandet sein. Dadurch kann ein direkter Kontakt zwischen dem Körper 2 und der Messeinheit 3 verhindert werden. Dadurch muss beispielsweise nicht darauf geachtet werden, dass die Messeinheit 3 selbst hautverträglich ist. Beispielsweise kann die Messeinheit 3 als Halbleiterchip ausgebildet sein. Das Material der Messeinheit 3 bzw. die Messeinheit 3 selbst kann nicht darauf geprüft sein, ob es gesundheitsgefährdend oder hautreizend ist. Mittels der hautverträglichen Schutzschicht 6 können auch ungeprüfte Messeinheiten 3 verwendet werden, da die Schutzschicht 6 die Messeinheit 3 abkapselt bzw. einen direkten Kontakt der Messeinheit 3 mit dem Körper 2 verhindert.

Die Schutzschicht 6 kann des Weiteren als eine Flüssigkeits- und/oder Gasdiffusionsbarriere ausgebildet sein. Dadurch kann beispielsweise eine Korrosion der Messeinheit 3 verhindert werden.

Die Schutzschicht 6 kann ferner beispielsweise wasserdicht ausgebildet sein, so dass ein Eindringen von Feuchtigkeit in die Messvorrichtung 1 verhindert werden kann. Die Schutzschicht 6 kann zusätzlich oder alternativ auch eine Dichtigkeit für Gase aufweisen bzw. gasdicht sein, so dass beispielsweise Gase bei einem Ausgasen von Materialien der Messvorrichtung 1 nicht in Kontakt mit dem Körper 2 kommen.

Gemäß dem vorliegenden Ausführungsbeispiel der Figur 1 ist die Schutzschicht 6 an der Unterseite 5 der Messvorrichtung 1 angeordnet.

Die Schutzschicht 6 kann ferner eine Dicke aufweisen, dass der Wärmefluss bzw. die Wärmeleitung zwischen dem Körper 2 und der Messeinheit 3 nur wenig beeinflusst ist. Beispielsweise weist die Schutzschicht 6 eine Dicke auf, die im Bereich zwischen 0,001 mm und 1 mm liegt. Die Dicke kann auch in einem Bereich zwischen 25 µm und 500 µm liegen. Die Dicke der Schutzschicht 6 kann aber auch 50 µm betragen. Die Schutzschicht 6 sollte so dünn wie möglich sein, so dass die Messeinheit 3 die Körpertemperatur gut messen kann.

Des Weiteren weist die Messvorrichtung 1 eine erste Klebeschicht 8 auf. Mit Hilfe der Klebeschicht 8 kann die Messvorrichtung 1 auf dem Körper 2 bzw. auf der Haut des Körpers 2 befestigt werden, so dass die Körpertemperatur des Körpers 2 beispielsweise über einen längeren Zeitraum und dadurch auch Änderungen in der Körpertemperatur ermittelt werden können.

Die erste Klebeschicht 8 ist beim verwendungsgemäßen Gebrauch der Messvorrichtung 1 an der dem Körper 2 zugewandten Seite der Messvorrichtung 1 angeordnet. Die erste Klebeschicht 8 ist an der Unterseite 5 der Messvorrichtung 1 angeordnet. Die hautverträgliche Schutzschicht 6 ist zwischen der ersten Klebeschicht 8 und der Messeinheit 3 angeordnet. Die erste Klebeschicht 8 erstreckt sich dabei flächig über die Unterseite 5 der Messvorrichtung 1, so dass die Messvorrichtung 1 sicher auf dem Körper 2 angeordnet werden kann. Die erste Klebeschicht 8 kann sich ferner vollständig über die hautverträgliche Schutzschicht 6 erstrecken. Die erste Klebeschicht 8 kann beispielsweise Kanäle aufweisen, um Schweiß zwischen dem Körper 2 und der Messvorrichtung 1 abtransportieren zu können. Die Messvorrichtung 1 kann ein hier nicht gezeigtes Drainagesystem in der ersten Klebeschicht 8 aufweisen, um den Schweiß zwischen dem Körper 2 und der Messvorrichtung 1 abzuleiten. Dieses Drainagesystem kann durch die Kanäle in der ersten Klebeschicht 8 ausgebildet sein.

Gemäß dem vorliegenden Ausführungsbeispiel weist die Messvorrichtung 1 eine zweite Klebeschicht 15 auf. Die zweite Klebeschicht 15 ist zwischen der hautverträglichen Schutzschicht 6 und der Messeinheit 3 angeordnet. Dadurch kann die hautverträgliche Schutzschicht 6 mit der Messeinheit 3 verbunden sein. Die hautverträgliche Schutzschicht 6 ist zwischen der ersten Klebeschicht 8 und der zweiten Klebeschicht 15 angeordnet.

Die erste Klebeschicht 8, die hautverträgliche Schutzschicht 6 und die zweite Klebeschicht 15 können als eine doppelseitig klebende hautverträgliche Schutzschicht ausgebildet sein. Die erste Klebeschicht 8, die hautverträgliche Schutzschicht 6 und die zweite Klebeschicht 15 können somit einteilig ausgebildet sein. Beispielsweise können die erste und die zweite Klebeschicht 8, 15 auf die hautverträgliche Schutzschicht 6 aufgetragen sein. Die erste Klebeschicht 8, die hautverträgliche Schutzschicht 6 und die zweite Klebeschicht 15 können die Gestalt eines doppelseitigen hautverträglichen Klebebandes aufweisen. Die erste Klebeschicht 8, die hautverträgliche Schutzschicht 6 und die zweite Klebeschicht 15 können einen Klebe-Schutzschicht-Verbund 18 bilden, welcher einteilig ausgebildet sein kann.

Die Schutzschicht 6 und/oder die Klebeschicht 8 können ferner eine plane Fläche bilden, so dass diese vollständig auf dem Körper 2 angelegt werden können.

Außerdem kann die Messvorrichtung 1 eine Versiegelung 7 aufweisen, welche beispielsweise aus einem Kunststoffschaum ausgebildet ist. Die Versiegelung 7 kann auch eine Vergießung sein. Mit Hilfe der Versiegelung 7 können die Messeinheit 3 versiegelt bzw. vergossen sein. Dadurch sind die Messeinheit 3 vor Beschädigung, vor Gasen und/oder vor Feuchtigkeit von außerhalb der Messvorrichtung 1 geschützt. Die Messeinheit 3 ist dadurch abgekapselt, so dass die Versiegelung 7 auch eine Verkapselung sein kann. Die Versiegelung 7 kann dabei aus einem flexiblen Material sein, so dass sich die Versiegelung 7 und somit die Messvorrichtung 1 an eine Oberflächenkontur des Körpers 2 anpassen kann.

Die Versiegelung 7 kann die Messeinheit 3 vollständig umschließen. Die Versiegelung 7 kann beispielsweise aus zumindest einer Schaumfolie ausgebildet sein. Die Schaumfolie kann ferner zumindest eine Aussparung aufweisen, in der zumindest die Messeinheit 3 und/oder eine Batterie als Energieeinheit 13 angeordnet sein kann. Die Schaumfolie kann beispielsweise aus Polyethylenschaum ausgebildet sein.

Vorteilhaft ist es, wenn die Versiegelung 7 wärmeisolierend und die Schutzschicht 6 und/oder die erste Klebeschicht 8 wärmeleitend ist. Insbesondere weist die Schutzschicht 6 und/oder die erste Klebeschicht 8 eine höhere Wärmeleitfähigkeit auf als die Versiegelung 7.

Die Versiegelung 7 kann auch aus einem Schaum, insbesondere einem Polyethylenschaum, ausgebildet sein. Ferner kann die Schutzschicht 6 aus Polyester ausgebildet sein. Eine derartige Materialkombination ist vorteilhaft, da Polyester im Vergleich zu dem Schaum, insbesondere dem Polyethylenschaum, eine bessere Wärmeleitfähigkeit aufweist. Es ist aber auch von Vorteil, dass der Schaum, insbesondere der Polyethylenschaum, eine im Vergleich zum Polyester schlechtere Wärmeleitfähigkeit aufweist. Die Schutzschicht 6 aus Polyester kann schnell die Körpertemperatur zur Messeinheit 3 leiten. Infolgedessen kann die Messeinheit 3 schnell die Körpertemperatur messen. Daneben wird durch die Versiegelung 7 aus dem Schaum, insbesondere dem Polyethylenschaum, die Messeinheit 3 von einer Umgebungstemperatur isoliert. Die Versiegelung 7 ist somit eine Isolationsschicht. Die Messeinheit 3 misst dadurch nicht die Umgebungstemperatur, sondern die Körpertemperatur. Eine Messgenauigkeit der Messung der Körpertemperatur ist dadurch verbessert.

Zusätzlich oder alternativ kann die erste und/oder die zweite Klebeschicht aus einem Acrylat-Copolymer ausgebildet sein, so dass auch diese im Vergleich zum Schaum die gute Wärmeleitfähig aufweisen. Die erste und/oder die zweite Klebeschicht behindern dadurch die Messung der Körpertemperatur ebenfalls wenig.

Zusätzlich oder alternativ sind die erste und/oder die zweite Klebeschicht so dünn wie möglich, beispielsweise zwischen 5 µm und 100 µm, ausgebildet.

Infolgedessen wird die Wärmeleitung zwischen dem Körper 2 und der Messeinheit 3 so wenig wie möglich behindert.

Die erste Klebeschicht 8, die Schutzschicht 6 und/oder die zweite Klebeschicht 15 erstrecken sich gemäß dem vorliegenden Ausführungsbeispiel vollständig über die Messeinheit 3 und/oder die Versiegelung 7. Gemäß dem vorliegenden Ausführungsbeispiel erstrecken sich die erste Klebeschicht 8, die Schutzschicht 6 und/oder die zweite Klebeschicht 15 seitlich über die Messeinheit 3 und/oder die Versiegelung 7 hinaus.

Gemäß dem vorliegenden Ausführungsbeispiel sind zwei Randbereiche 20a, 20b gezeigt. Die Randbereiche 20a, 20b können auch vollständig um die Messeinheit 3 und/oder die Versiegelung 7 laufen, so dass die Randbereich 20a, 20b miteinander verbunden sind und ineinander übergehen.

Gemäß dem vorliegenden Ausführungsbeispiel weist die Messvorrichtung 1 eine äußere Klebeschicht 17 auf. Diese ist beim verwendungsgemäßen Gebrauch der Messvorrichtung 1 auf der dem Körper 2 abgewandten Seite der Messvorrichtung 1 angeordnet. Die äußere Klebeschicht 17 ist somit an der Oberseite 10 der Messvorrichtung 1 angeordnet. Die äußere Klebeschicht 17 kann zumindest die Messeinheit 3 von der Umgebung der Messvorrichtung 1 abkapseln. Dabei kann die äußere Klebeschicht 17 beispielsweise wie der Klebe-Schutzschicht-Verbund 18 ausgebildet sein. Insbesondere kann die äußere Klebeschicht 17 eine hautverträgliche Schutzschicht und zumindest eine, insbesondere zwei, Klebeschichten aufweisen. Die äußere Klebeschicht 17 und der Klebe-Schutzschicht-Verbund 18 und/oder die zweite Klebeschicht 15 können insbesondere in deren Randbereichen 20a, 20b miteinander verklebt sein, so dass die Messeinheit 3 vollständig eingekapselt ist. Die Randbereiche 20a, 20b erstrecken sich, wie hier gezeigt ist, seitlich weg, so dass diese die Messeinheit 3 und/oder die Versiegelung 7 zumindest teilweise, insbesondere vollständig, umranden.

Beispielsweise kann die äußere Klebeschicht 17 mit der zweiten Klebeschicht 15 zusammengeklebt sein, so dass diese beiden Klebeschichten 15, 17 zumindest die Messeinheit 3 und/oder die Versiegelung 7 einkapseln. Beispielsweise kann die äußere Klebeschicht 17 mit der zweiten Klebeschicht 15 in Randbereichen 20a, 20b zusammengeklebt sein.

Gemäß dem vorliegenden Ausführungsbeispiel weist die Messvorrichtung 1 eine Außenschicht 16 auf, die beispielsweise als Textilschicht ausgebildet sein kann. Die Außenschicht 16 kann beispielsweise auf die äußere Klebeschicht 17 aufgeklebt sein. Mit Hilfe der Außenschicht 16 kann ein Tragekomfort der Messvorrichtung 1 verbessert werden. Die Außenschicht 16 kann sich ferner, insbesondere in einer Querrichtung der Messvorrichtung 1, über den Klebe-Schutzschicht-Verbund 18, den erste Klebeschicht 8, die hautverträgliche Schutzschicht 6, die zweite Klebeschicht 15 und/oder die äußere Klebeschicht 17 hinaus erstrecken.

Gemäß dem vorliegenden Ausführungsbeispiel ist die äußere Klebeschicht 17 vollflächig auf der Außenschicht 16 angeordnet. Dadurch kann die Außenschicht 16 mit Hilfe der äußeren Klebeschicht 17 auf den Körper 2 geklebt werden. Die Messvorrichtung 1 ist dadurch mit Hilfe der äußeren Klebeschicht 17 sicher auf den Körper 2 geklebt. Die Außenschicht 16 ist mit Hilfe der äußeren Klebeschicht 17 ebenfalls am Körper 2 angeklebt.

Die Außenschicht 16 und die äußere Klebeschicht 17 können einen Textil-Klebe-Verbund bilden.

Im vorliegenden Ausführungsbeispiel erstreckt sich die Außenschicht 16 und die äußere Klebeschicht 17 über die Schutzschicht 6 und/oder die erste Klebeschicht 8 hinweg. In den Randbereichen 20a, 20b sind die Außenschicht 16, die äußere Klebeschicht 17 und die Schutzschicht 6 und/oder die erste Klebeschicht 8 miteinander verbunden. In den Randbereichen 20a, 20b kann die Messvorrichtung 1 flächig auf dem Körper 2 aufgeklebt werden. Die Randbereiche 20a, 20b können dann eine Barriere für Wasser und/oder Feuchtigkeit bilden. Die Randbereiche 20a, 20b, wenn diese auf dem Körper 2 aufgeklebt sind, können verhindern, dass Wasser oder Feuchtigkeit und insbesondere Schmutz in den Bereich zwischen Messeinheit 3 und Körper 2 gelangt.

In den Randbereichen 20a, 20b ragen die Außenschicht 16 und/oder die äu-ßere Klebeschicht 17 auch seitlich über die Schutzschicht 6, die erste und/oder die zweite Klebeschicht 8, 15 hinaus. Die äußere Klebeschicht 17 und/oder die Außenschicht 16 kleben somit direkt auf dem Körper 2. Ferner ist es von Vorteil, wenn die Außenschicht 16 und/oder die äußere Klebeschicht 17 luft- und/oder wasserdurchlässig ist, so dass dadurch in diesen Bereichen, insbesondere in den Randbereichen 20a, 20b, beispielsweise Schweiß verdunsten kann, so dass ein Tragekomfort verbessert ist.

Wie hier gezeigt ist, überlappen in den Randbereichen 20a, 20b die äußere Klebeschicht 17 und die zweite Klebeschicht 15 zumindest bereichsweise. Dort sind beide Schichten 15, 17 zusammengeklebt, so dass zumindest die Messeinheit 3 und/oder die Versiegelung 7 eingekapselt ist. Außerdem ist dadurch eine Verbindung mit der Schutzschicht 6, der ersten Klebeschicht 8 und der zweiten Klebeschicht 15 zur äußeren Klebeschicht 17 und der Au-ßenschicht 16 gegeben.

Wenn die Außenschicht 16, die äußere Klebeschicht 17 und die Schutzschicht 6 und/oder die erste Klebeschicht 8 miteinander verbunden sind, weist dies den Vorteil auf, dass infolgedessen die Messeinheit 3, die Versiegelung 7, eine Trägereinheit 9, ein Wärmeleitelement 11, eine Energieeinheit 13 und/oder eine Schnittstelle 14 eingekapselt ist. Die Außenschicht 16, die äußere Klebeschicht 17 und die Schutzschicht 6 und/oder die erste Klebeschicht 8 bilden somit eine Umhüllung für zumindest die Messeinheit 3 und/oder die Versiegelung 7.

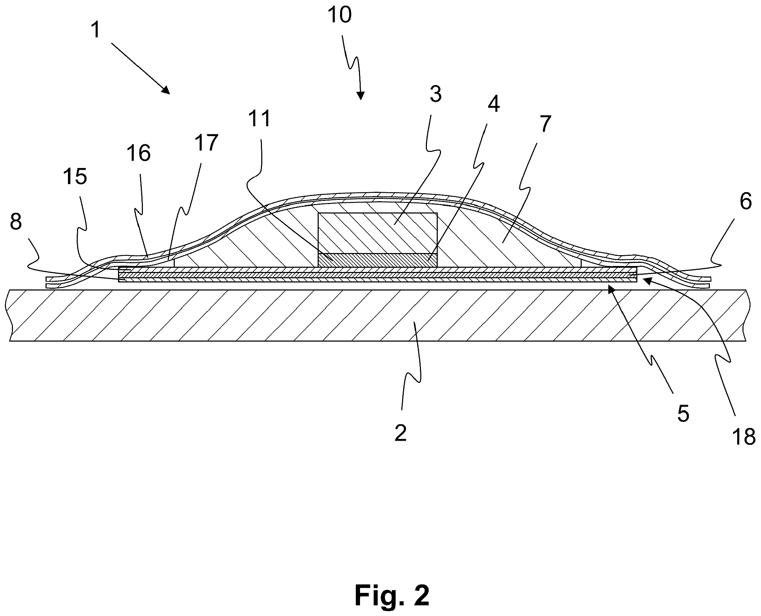

Figur 2 zeigt eine schematische Schnittansicht einer Messvorrichtung 1 mit der Messeinheit 3, einem Wärmeleitelement 4, der Schutzschicht 6 und der ersten Klebeschicht 8.

Merkmale, die bereits in der vorangegangenen Figur beschrieben sind, werden der Einfachheit halber nicht nochmals beschrieben. Ferner werden für gleiche oder zumindest ähnliche Merkmale gleiche Bezugszeichen verwendet. Der Übersichtlichkeit halber können Merkmale auch erst in einer oder mehreren folgenden Figuren beschrieben werden. Es werden in den folgenden Figuren für gleiche oder ähnliche Merkmale ebenfalls gleiche Bezugszeichen verwendet.

Um die Temperatur der Messeinheit 3 an die Körpertemperatur des Körpers 2 anzupassen, so dass die Körpertemperatur durch die Messeinheit 3 ermittelt werden kann, weist die Messvorrichtung 1 das Wärmeleitelement 4 auf. Mittels des Wärmeleitelements 4 kann ein Wärmefluss zwischen dem Körper 2 und der Messeinheit 3 ausgebildet werden. Dabei kann der Wärmefluss bidirektional zwischen der Messeinheit 3 und dem Körper 2 ausgebildet sein.

Weist beispielsweise der Körper 2 eine höhere Temperatur auf als die Messeinheit 3, leitet das Wärmeleitelement 4 Wärme vom Körper 2 zur Messeinheit 3, bis die Temperaturen ausgeglichen sind.

Weist dagegen der Körper 2 eine niedrigere Temperatur auf als die Messeinheit 3, leitet das Wärmeleitelement 4 Wärme von der Messeinheit 3 zum Körper 2, bis die Temperaturen ausgeglichen sind.

Sind die Temperaturen ausgeglichen, kann die Körpertemperatur des Körpers 2 ermittelt werden.

Das Wärmeleitelement 4 ist hier flächig ausgebildet. Das Wärmeleitelement 4 kann ein Flächenelement 11 umfassen. Das Wärmeleitelement 4 kann als das Flächenelement 11 ausgebildet sein. Durch das Wärmeleitelement 4 bzw. das Flächenelement 11 kann die Körpertemperatur schnell gemessen werden, da die Wärme schnell zwischen der Messeinheit 3 und dem Körper 2 ausgetauscht werden kann.

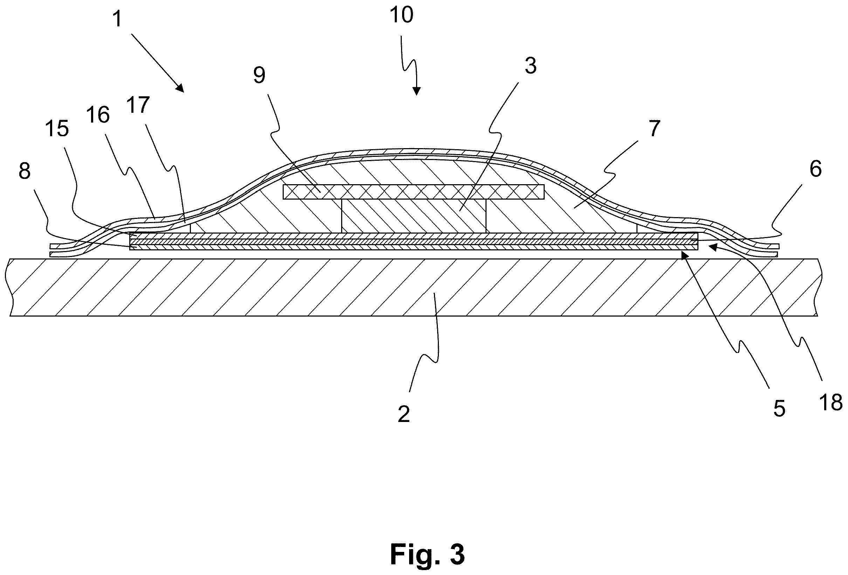

Figur 3 zeigt eine Messvorrichtung 1 mit einer Trägereinheit 9. Die Trägereinheit 9 kann beispielsweise eine Leiterplatte sein, die vorzugsweise flexibel ausgebildet ist. Die Leiterplatte kann Leiterbahnen umfassen, wobei die Messeinheit 3 mit der Leiterplatte elektrische leitend verbunden ist. Die Leiterplatte kann beispielsweise die Messeinheit 3 mit elektrischer Energie versorgen. Zusätzlich oder alternativ können die Messwerte zur Körpertemperatur über die Leiterplatte an eine Schnittstelle, beispielsweise eine Antenne, leiten, so dass die Messwerte zur Körpertemperatur von einer externen Ausleseeinheit ausgewertet werden können.

Merkmale, die bereits in einer der vorangegangenen Figuren beschrieben sind, werden der Einfachheit halber nicht nochmals beschrieben. Ferner werden für gleiche oder zumindest ähnliche Merkmale gleiche Bezugszeichen verwendet. Der Übersichtlichkeit halber können Merkmale auch erst in einer oder mehreren folgenden Figuren beschrieben werden. Es werden in den folgenden Figuren für gleiche oder ähnliche Merkmale ebenfalls gleiche Bezugszeichen verwendet.

Die Messeinheit 3 ist im vorliegenden Ausführungsbeispiel an der beim verwendungsgemäßen Gebrauch dem Körper 2 zugewandten Seite der Trägereinheit 9 angeordnet.

Die Trägereinheit 9 kann auch ein Grundgerüst für die Messvorrichtung 1 ausbilden.

Mit Hilfe der Trägereinheit 9 kann die Messvorrichtung 1 stabiler ausgebildet werden. Dabei kann die Trägereinheit 9 aus einem flexiblen Material ausgebildet sein, so dass sich die Trägereinheit 9 an die Körperkontur anpassen kann. Die Trägereinheit 9 kann beispielsweise aus einem elastischen Kunststoff hergestellt sein.

Die Messeinheit 3 und die Trägereinheit 9 sind im vorliegenden Ausführungsbeispiel vollständig von der Versiegelung 7 umschlossen.

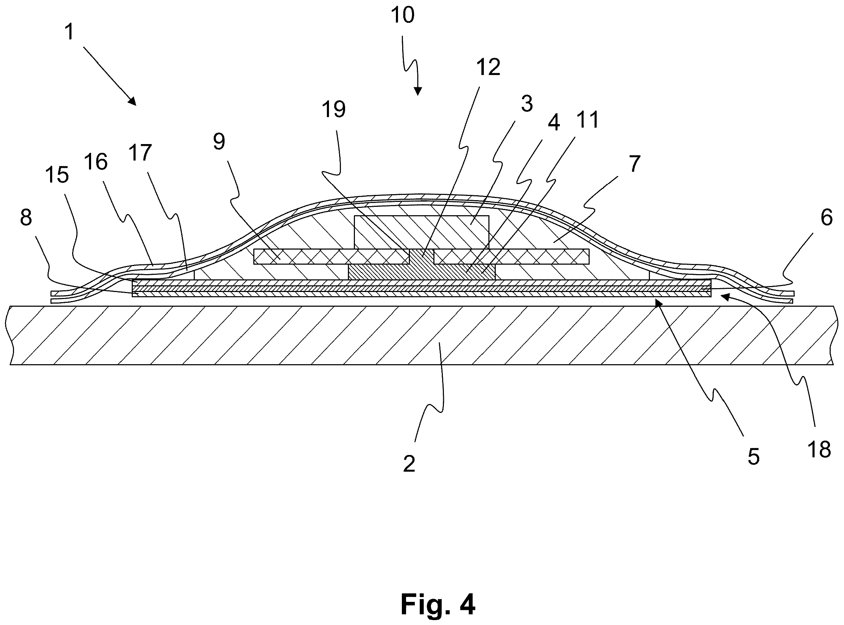

Figur 4 zeigt eine Messvorrichtung 1 mit der Trägereinheit 9 und einer Durchkontaktierung 12. Merkmale, die bereits in einer der vorangegangenen Figuren beschrieben sind, werden der Einfachheit halber nicht nochmals beschrieben. Ferner werden für gleiche oder zumindest ähnliche Merkmale gleiche Bezugszeichen verwendet. Der Übersichtlichkeit halber können Merkmale auch erst in einer oder mehreren folgenden Figuren beschrieben werden. Es werden in den folgenden Figuren für gleiche oder ähnliche Merkmale ebenfalls gleiche Bezugszeichen verwendet.

Im vorliegenden Ausführungsbeispiel ist die Messeinheit 3 auf der beim verwendungsgemäßen Gebrauch der Messvorrichtung 1 dem Körper 2 abgewandten Seite der Trägereinheit 9 angeordnet. Die Messvorrichtung 1 weist das Wärmeleitelement 4 auf, welches in diesem Ausführungsbeispiel das Flächenelement 11 aufweist, das auf der zur Messeinheit 3 abgewandten Seite der Trägereinheit 9 angeordnet ist. Das Wärmeleitelement 4 weist ferner eine Durchkontaktierung 12 auf, welche vom Flächenelement 11 durch die Trägereinheit 9 hindurch zur Messeinheit 3 führt. Die Trägereinheit 9 kann einen Kontaktierungsdurchbruch 19 aufweisen, durch den sich die Durchkontaktierung 12 hindurcherstreckt. Der Kontaktierungsdurchbruch 19 kann beispielsweise einen runden Querschnitt aufweisen. Mit Hilfe der Durchkontaktierung 12 kann der Wärmefluss durch die Trägereinheit 9 ausgebildet werden. Dadurch kann die Messeinheit 3 auf der einen Seite der Trägereinheit 9 und das Flächenelement 11 auf der dazu gegenüberliegenden Seite der Trägereinheit 9 angeordnet sein.

Das Wärmeleitelement 4 weist im vorliegenden Ausführungsbeispiel der Figur 2 die Durchkontaktierung 12 auf. Die Durchkontaktierung 12 erstreckt sich durch die Trägereinheit 9 hindurch. Die Durchkontaktierung 12 erstreckt sich ferner von der Messeinheit 3 zur Schutzschicht 6, um den Wärmefluss zwischen dem Körper 2 und der Messeinheit 3 auszubilden.

Zusätzlich oder alternativ kann, wie in diesem Ausführungsbeispiel der Figur 4 gezeigt ist, das Wärmeleitelement 4 auch das Flächenelement 11 aufweisen. Das Wärmeleitelement 4 kann somit die Durchkontaktierung 12 und das Flächenelement 11 umfassen. Die Durchkontaktierung 12 erstreckt sich somit von der Messeinheit 3, durch die Trägereinheit 9 zum Flächenelement 11. Die Durchkontaktierung 12 ist zwischen der Messeinheit 3 und dem Flächenelement 11 angeordnet. Die Durchkontaktierung 12 leitet die Wärme vom Flächenelement 11 zur Messeinheit 3 weiter, wenn die Körpertemperatur höher ist als die Temperatur der Messeinheit 3. Alternativ kann die Durchkontaktierung 12 die Wärme von der Messeinheit 3 zum Flächenelement 11 leiten, wenn die Körpertemperatur des Körpers 2 niedriger ist als die Temperatur der Messeinheit 3.

An die Trägereinheit 9 kann ferner die Messeinheit 3, die Durchkontaktierung 12 und/oder das Flächenelement 11 angeordnet sein, so dass die Messvorrichtung 1 insgesamt stabiler ist. Zusätzlich oder alternativ kann auch das Wärmeleitelement 4 an der Trägereinheit 9 angeordnet sein.

Zusätzlich oder alternativ kann auch die Versiegelung 7 mit der Trägereinheit 9 verbunden sein, so dass die Messvorrichtung 1 insgesamt stabiler ist. Gemäß dem vorliegenden Ausführungsbeispiel kann die Versiegelung 7 zumindest teilweise um die Trägereinheit 9 angeordnet sein.

Gemäß dem vorliegenden Ausführungsbeispiel weist die Durchkontaktierung 12 einen geringeren Querschnitt auf als das Flächenelement 11.

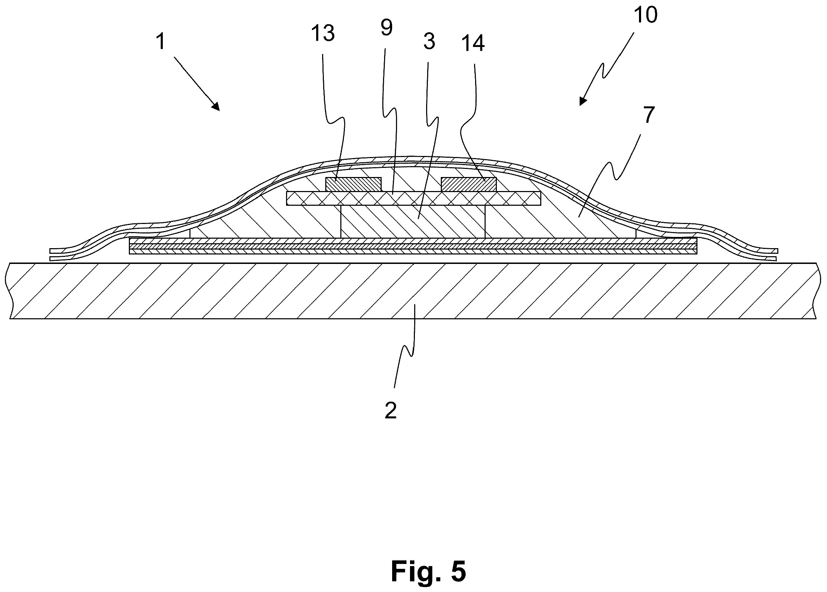

Figur 5 zeigt eine schematische Schnittansicht einer Messvorrichtung 1 zum Messen einer Körpertemperatur mit einer Energieeinheit 13 und einer Schnittstelle 14. In der hier gezeigten Figur sind lediglich die wichtigsten und neuen Merkmale mit einem Bezugszeichen versehen.

Merkmale, die bereits in einer der vorangegangenen Figuren beschrieben sind, werden der Einfachheit halber nicht nochmals beschrieben. Ferner werden für gleiche oder zumindest ähnliche Merkmale gleiche Bezugszeichen verwendet. Der Übersichtlichkeit halber können Merkmale auch erst in einer oder mehreren folgenden Figuren beschrieben werden. Es werden in den folgenden Figuren für gleiche oder ähnliche Merkmale ebenfalls gleiche Bezugszeichen verwendet.

Die Energieeinheit 13 kann beispielsweise eine Batterie, eine Akkumulator und/oder einen Superkondensator umfassen, mit der zumindest die Messeinheit 3 mit elektrischer Energie versorgt werden kann. Die Energieeinheit 13 kann aber auch ein Piezoelement umfassen, das auch eine Bewegung, beispielsweise Biegung, der Messvorrichtung 1 am Körper 2 elektrische Energie erzeugt. Die Energieeinheit 13 kann aber auch ein Peltierelement umfassen, welches aus einer Temperaturdifferenz, insbesondere zwischen dem Körper 2 und einer Oberseite 10 der Messvorrichtung 1, elektrische Energie erzeugt. Zusätzlich oder alternativ kann die Energieeinheit 13 eine kapazitive und/oder induktive Einheit umfassen, welche aus einem elektrischen und/oder magnetischen Feld elektrische Energie erzeugt.

Zusätzlich oder alternativ weist die Messvorrichtung 1 die Schnittstelle 14 auf, mittels der die ermittelte Körpertemperatur des Körpers 2 beispielsweise ausgelesen werden kann. Die Schnittstelle 14 kann beispielsweise eine hier nicht gezeigte Antenneneinheit aufweisen, mittels der die Körpertemperatur über Funk ausgelesen werden kann. An die Schnittstelle 14 kann sich beispielsweise ein geeignetes Auslesegerät ankoppeln, um die Körpertemperatur auszulesen. Beispielsweise umfasst die Schnittstelle 14 eine RFID-Schnittstelle. Die Schnittstelle 14 kann auch auf NFC-Technologie (near field communication) basieren.

Die Energieeinheit 13 und/oder die Schnittstelle 14 können beispielsweise mittels der Trägereinheit 9 mit der Messeinheit 3 verbunden sein. Dabei ist es vorteilhaft, wenn die Trägereinheit 9 als, insbesondere flexible, Leiterplatte ausgebildet ist, die die elektrische Verbindung zwischen der Messeinheit 3 und der Energieeinheit 13 und/oder der Schnittstelle 14 ausbilden kann.

Die in den Figuren gezeigten schematischen Schnittansichten sind dabei nicht maßstabsgetreu dargestellt. Beispielsweise können die Schichtdicken der Schutzschicht 6, der ersten Klebeschicht 8, der zweiten Klebeschicht 15, der Außenschicht 16 und/oder der äußeren Klebeschicht 17 natürlich, insbesondere im Verhältnis zur Messvorrichtung 1, dünner ausgebildet sein.

Ferner können alle elektrischen Bauteile, wie beispielsweise die Messeinheit 3, die Energieeinheit 13 und/oder die Schnittstelle 14, von einem Lack abgekapselt sein, welcher wasser- und/oder gasdicht ist. Der Lack kann des Weiteren eine hohe Flüssigkeits- und/oder Gasdiffusionsbarriere ausbilden.

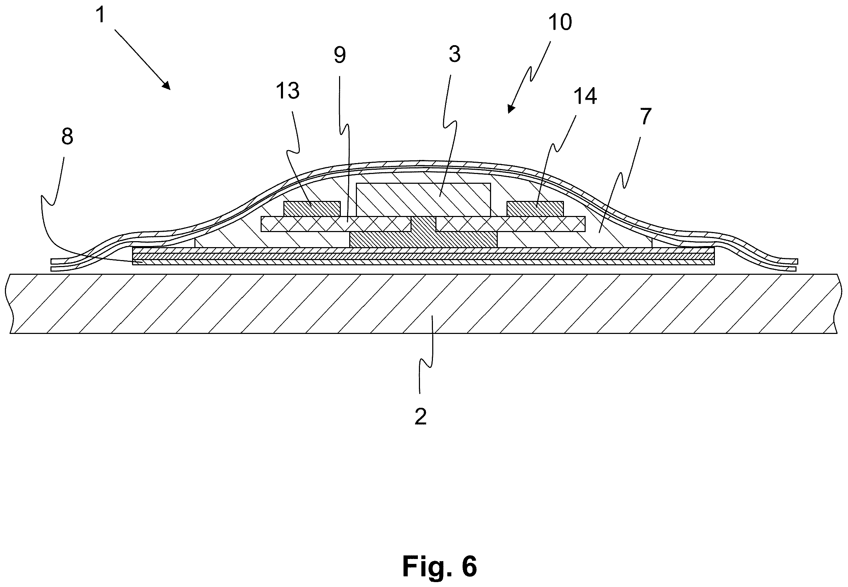

Figur 6 zeigt ein schematisches Ausführungsbeispiel einer Messvorrichtung 1 mit einer Messeinheit 3, der Energieeinheit 13 und/oder der Schnittstelle 14.

Merkmale, die bereits in einer der vorangegangenen Figuren beschrieben sind, werden der Einfachheit halber nicht nochmals beschrieben. Ferner werden für gleiche oder zumindest ähnliche Merkmale gleiche Bezugszeichen verwendet. Der Übersichtlichkeit halber können Merkmale auch erst in einer oder mehreren folgenden Figuren beschrieben werden. Es werden in den folgenden Figuren für gleiche oder ähnliche Merkmale ebenfalls gleiche Bezugszeichen verwendet.

Der Einfachheit halber sind lediglich die zum Verständnis wichtigsten Merkmale mit einem Bezugszeichen versehen. Gemäß dem vorliegenden Ausführungsbeispiel ist die Energieeinheit 13 und/oder die Schnittstelle 14 mit der Messeinheit 3 auf der zur ersten Klebeschicht 8 abgewandten Seite der Trägereinheit 9 angeordnet. Zwischen der Energieeinheit 13, der Schnittstelle 14 und/oder der Messeinheit 3 und der ersten Klebeschicht 8 ist zumindest die Trägereinheit 9 angeordnet. Dadurch ist ein Abstand zwischen der Energieeinheit 13 und/oder der Schnittstelle 14 und der Messeinheit 3 minimiert, so dass elektrische Leitungen zwischen der Messeinheit 3 und der Energieeinheit 13 und/oder der Schnittstelle 14 kurz gehalten sind. Beispielsweise kann die Energieeinheit 13 und/oder die Schnittstelle 14 mit der Messeinheit 3 in direktem Kontakt stehen. Wenn die Energieeinheit 13 und/oder die Schnittstelle 14 sowie die Messeinheit 3 auf einer Seite der Trägereinheit 9 angeordnet sind, bringt dies einen weiteren Vorteil mit sich. Wenn die Trägereinheit 9 gedruckte Leitungen aufweist oder selbst als gedruckte Leiterplatte ausgebildet ist, können die elektrischen, gedruckten Leitungen lediglich auf einer Seite angeordnet sein. Dadurch kann die Trägereinheit 9, wenn diese elektrische Leitungen aufweist oder als Leiterplatte ausgebildet ist, vereinfacht werden.

Gemäß dem vorliegenden Ausführungsbeispiel ist die Trägereinheit 9, die Messeinheit 3, die Energieeinheit 13 und die Schnittstelle 14 vollständig von der Versiegelung umschlossen.

Die vorliegende Erfindung ist nicht auf die dargestellten und beschriebenen Ausführungsbeispiele beschränkt. Abwandlungen im Rahmen der Patentansprüche sind ebenso möglich wie eine Kombination der Merkmale, auch wenn diese in unterschiedlichen Ausführungsbeispielen dargestellt und beschrieben sind.

### Bezugszeichenliste

- 1: Messvorrichtung
- 2: Körper
- 3: Messeinheit
- 4: Wärmeleitelement
- 5: Unterseite der Messvorrichtung
- 6: Schutzschicht
- 7: Versiegelung
- 8: erste Klebeschicht
- 9: Trägereinheit
- 10: Oberseite der Messvorrichtung
- 11: Flächenelement
- 12: Durchkontaktierung
- 13: Energieeinheit
- 14: Schnittstelle
- 15: zweite Klebeschicht
- 16: Außenschicht
- 17: äußere Klebeschicht
- 18: Klebe-Schutzschicht-Verbund
- 19: Kontaktierungsdurchbruch
- 20: Randbereiche

## Patentansprüche

1. Messvorrichtung (1) zum Messen einer Körpertemperatur eines Lebewesens, insbesondere eines Menschen,
mit einer Messeinheit (3) zum Messen der Körpertemperatur,
mit einem Wärmeleitelement (4), mittels dem beim bestimmungsgemäßen Gebrauch der Messvorrichtung (1) ein Wärmefluss zwischen einem Körper (2) des Lebewesens und der Messeinheit (3) ausbildbar ist,
mit einer Außenschicht (16), die an einer beim verwendungsgemäßen Gebrauch dem Körper gegenüberliegenden Oberseite der Messvorrichtung angeordnet ist und die auf eine äußere Klebeschicht (17) der Messvorrichtung (1) aufgeklebt ist, und
mit einer ersten Klebeschicht (8) zum Befestigen der Messvorrichtung (1) auf dem Körper (2) des Lebewesens, wobei die erste Klebeschicht (8) an einer dem dafür vorgesehenen Körper (2) zugewandten Seite der Messvorrichtung (1) angeordnet ist,
wobei das Wärmeleitelement (4) aus Metall ausgebildet ist,
die Messvorrichtung (1) eine hautverträgliche Schutzschicht (6) aufweist,
**dadurch gekennzeichnet, dass**
die hautverträgliche Schutzschicht (6) zwischen der ersten Klebeschicht (8) und der Messeinheit (3) und
das Wärmeleitelement (4) zwischen der hautverträglichen Schutzschicht (6) und der Messeinheit (3) angeordnet ist, und
dass die Außenschicht (16) und die äußere Klebeschicht (17) in Randbereichen (20a, 20b) der Messvorrichtung (1) seitlich über die Schutzschicht (6) und die erste Klebeschicht (8) hinausragen.

2. Messvorrichtung nach dem vorherigen Anspruch, **dadurch gekennzeichnet, dass** die zwischen der hautverträglichen Schutzschicht (6) und der Messeinheit (3) und/oder dem Wärmeleitelement (4) eine zweite Klebeschicht (15) angeordnet ist.

3. Messvorrichtung nach einem der vorherigen Ansprüche, **dadurch gekennzeichnet, dass** die Messvorrichtung (1) eine Trägereinheit (9) aufweist, in und/oder an der die Messeinheit (3) und/oder das Wärmeleitelement (4) angeordnet ist.

4. Messvorrichtung nach einem der vorherigen Ansprüche, **dadurch gekennzeichnet, dass** das Wärmeleitelement (4) ein Flächenelement (11) umfasst, das auf einer beim bestimmungsgemäßen Gebrauch der Messvorrichtung (1) dem Körper (2) zugewandten Seite der Messeinheit (3) angeordnet ist.

5. Messvorrichtung nach dem vorherigen Anspruch 4, **dadurch gekennzeichnet, dass** das Wärmeleitelement (4) eine Durchkontaktierung (12) umfasst, die zwischen dem Flächenelement (11) und der Messeinheit (3) angeordnet ist, so dass das Flächenelement (11) von der Messeinheit (3) beabstandet ist.

6. Messvorrichtung nach dem vorherigen Anspruch 5, **dadurch gekennzeichnet, dass** das Flächenelement (11) einen größeren Querschnitt als die Durchkontaktierung (12) aufweist.

7. Messvorrichtung nach dem vorherigen Anspruch 2, **dadurch gekennzeichnet, dass** sich die Schutzschicht (6), die erste und/oder die zweite Klebeschicht (8,15) vollständig über die im bestimmungsgemäßen Gebrauch dem Körper (2) zugewandte Seite der Messvorrichtung (1) erstreckt.

8. Messvorrichtung nach einem der vorherigen Ansprüche, **dadurch gekennzeichnet, dass** die Schutzschicht (6) und/oder die Klebeschicht (8) zumindest im Bereich des Wärmeleitelements (4) zumindest ein Wärmekontaktelement und/oder Zusatzstoffe aufweist, mittels denen der Wärmefluss erhöht werden kann.

9. Messvorrichtung nach einem der vorherigen Ansprüche, **dadurch gekennzeichnet, dass** die Messvorrichtung (1) eine Versiegelung (7) aufweist.

10. Messvorrichtung nach dem vorherigen Anspruch 9, **dadurch gekennzeichnet, dass** die Versiegelung (7) und die Schutzschicht (6) aus demselben Material sind.

11. Messvorrichtung nach einem der vorherigen Ansprüche 4 bis 6, **dadurch gekennzeichnet, dass** die Durchkontaktierung (12) und/oder das Flächenelement (11) aus Metall ausgebildet ist.

12. Messvorrichtung nach dem vorherigen Anspruch 3, **dadurch gekennzeichnet, dass** die Messvorrichtung (1) eine Energieeinheit (13), eine Speichereinheit und/oder Schnittstelle (14), insbesondere eine NFC-Schnittstelle, aufweist, die in und/oder an der Trägereinheit (9) und/oder der Messeinheit (3) angeordnet sind.

13. Messvorrichtung nach einem der vorherigen Ansprüche, **dadurch gekennzeichnet, dass** die Schutzschicht (6) aus Polyester und/oder Polyethylen ausgebildet ist.

## Claims

1. A measuring device (1) for measuring a body temperature of a living being, in particular of a human being,
with a measuring unit (3) for measuring the body temperature,
having a heat-conducting element (4), by means of which a heat flow is formable between a body (2) of the living being and the measuring unit (3) during the use of the measuring device (1) as intended having an outer layer (16), which is arranged on an upper side of the measuring device opposite to the body during use as intended, and which is adhered to an outer adhesive layer (17) of the measuring device (1), and
having a first adhesive layer (8) for fastening the measuring device (1) on the body (2) of the living being, wherein the first adhesive layer (8) is arranged at a side of the measuring device (1) facing the body (2) intended therefor,
wherein
the heat-conducting element (4) is made of metal,
the measuring device (1) includes a skin-compatible protective layer (6),
**characterized in that**
the skin-compatible protective layer (6) is arranged between the first adhesive layer (8) and the measuring unit (3) and
the heat-conducting element (4) is arranged between the skin-compatible protective layer (6) and the measuring unit (3), and
the outer layer (16) and the outer adhesive layer (17) project laterally beyond the protective layer (6) and the first adhesive layer (8) in edge regions (20a, 20b) of the measuring device (1).

2. The measuring device of the preceding claim, **characterized in that** a second adhesive layer (15) is arranged between the skin-compatible protective layer (6) and the measuring unit (3) and/or the heat-conducting element (4).

3. The measuring device of one of the preceding claims, **characterized in that** the measuring device (1) includes a carrier unit (9), in and/or at which the measuring unit (3) and/or the heat-conducting element (4) are/is arranged.

4. The measuring device of one of the preceding claims, **characterized in that** the heat-conducting element (4) includes a planar element (11), which is arranged on a side of the measuring unit (3) facing the body (2) during the use of the measuring device (1) as intended.

5. The measuring device of the preceding claim 4, **characterized in that** the heat-conducting element (4) includes a via (12), which is arranged between the planar element (11) and the measuring unit (3), and so the planar element (11) is spaced apart from the measuring unit (3).

6. The measuring device of the preceding claim 5, **characterized in that** the planar element (11) has a larger cross-section than the via (12).

7. The measuring device of the preceding claim 2, **characterized in that** the protective layer (6), the first adhesive layer (8) and/or the second adhesive layer (15) extend(s) completely across the side of the measuring device (1) facing the body (2) during the use as intended.

8. The measuring device of one of the preceding claims, **characterized in that** the protective layer (6) and/or the adhesive layer (8) include(s), at least in the area of the heat-conducting element (4), at least one thermal contact element and/or additives, by means of which the heat flow can be increased.

9. The measuring device of one of the preceding claims, **characterized in that** the measuring device (1) includes a sealing (7).

10. The measuring device of the preceding claim 9, **characterized in that** the sealing (7) and the protective layer (6) are made of the same material.

11. The measuring device of one of the preceding claims 4 to 6, **characterized in that** the via (12) and/or the planar element (11), are/is made of metal.

12. The measuring device of the preceding claim 3, **characterized in that** the measuring device (1) includes an energy unit (13), a memory unit and/or interface (14), in particular an NFC interface, which are arranged in and/or at the carrier unit (9) and/or the measuring unit (3).

13. The measuring device of one of the preceding claims, **characterized in that** the protective layer (6) is made of polyester and/or polyethylene.

## Revendications

1. Dispositif de mesure (1) pour mesurer une température du corps d'un être vivant, en particulier d'un être humain,
avec une unité de mesure (3) pour mesurer la température du corps,
avec un élément conducteur de chaleur (4), au moyen duquel, lors de l'usage conforme du dispositif de mesure (1), un flux de chaleur peut être formé entre un corps (2) de l'être vivant et l'unité de mesure (3),
avec une couche extérieure (16) qui est disposée sur une face supérieure du dispositif de mesure opposée au corps lors de l'usage conforme à la destination et qui est collée sur une couche adhésive extérieure (17) du dispositif de mesure (1), et
avec une première couche adhésive (8) pour fixer le dispositif de mesure (1) sur le corps (2) de l'être vivant, la première couche adhésive (8) étant disposée sur un côté du dispositif de mesure (1) orienté vers le corps (2) prévu à cet effet,
l'élément conducteur de chaleur (4) étant réalisé en métal,
le dispositif de mesure (1) présentant une couche de protection (6) à bonne affinité cutanée,
**caractérisé en ce que**
la couche de protection (6) à bonne affinité cutanée est disposée entre la première couche adhésive (8) et l'unité de mesure (3), et
l'élément conducteur de chaleur (4) est disposé entre la couche de protection (6) à bonne affinité cutanée et l'unité de mesure (3), et
**en ce que** la couche extérieure (16) et la couche adhésive extérieure (17) dépassent latéralement sur la couche de protection (6) et la première couche adhésive (8) dans les zones de bordure (20a, 20b) du dispositif de mesure (1).

2. Dispositif de mesure selon la revendication précédente, caractérisé en ce qu'une deuxième couche adhésive (15) est disposée entre la couche de protection (6) à bonne affinité cutanée et l'unité de mesure (3) et/ou l'élément conducteur de chaleur (4).

3. Dispositif de mesure selon l'une quelconque des revendications précédentes, **caractérisé en ce que** le dispositif de mesure (1) présente une unité de support (9) dans et/ou sur laquelle sont/est disposé(e)(s) l'unité de mesure (3) et/ou l'élément conducteur de chaleur (4).

4. Dispositif de mesure selon l'une quelconque des revendications précédentes, **caractérisé en ce que** l'élément conducteur de chaleur (4) comprend un élément plan (11) qui est disposé sur un côté de l'unité de mesure (3) qui est orienté vers le corps (2) lors de l'usage conforme du dispositif de mesure (1).

5. Dispositif de mesure selon la revendication 4 précédente, **caractérisé en ce que** l'élément conducteur de chaleur (4) comprend un trou d'interconnexion (12) qui est disposé entre l'élément plan (11) et l'unité de mesure (3), de sorte que l'élément plan (11) est à distance de l'unité de mesure (3).

6. Dispositif de mesure selon la revendication 5 précédente, **caractérisé en ce que** l'élément plan (11) présente une section transversale supérieure à celle du trou d'interconnexion (12).

7. Dispositif de mesure selon la revendication 2 précédente, **caractérisé en ce que** la couche de protection (6), la première et/ou la deuxième couche(s) adhésive(s) (8, 15) s'étend(ent) entièrement sur le côté du dispositif de mesure (1) qui est orienté vers le corps (2) lors de l'usage conforme.

8. Dispositif de mesure selon l'une quelconque des revendications précédentes, **caractérisé en ce que** la couche de protection (6) et/ou la couche adhésive (8) présente(nt) au moins dans la zone de l'élément conducteur de chaleur (4) au moins un élément de contact thermique et/ou des additifs au moyen desquels le flux de chaleur peut être augmenté.

9. Dispositif de mesure selon l'une quelconque des revendications précédentes, **caractérisé en ce que** le dispositif de mesure (1) comporte une imperméabilisation (7).

10. Dispositif de mesure selon la revendication 9 précédente, **caractérisé en ce que** l'imperméabilisation (7) et la couche de protection (6) sont réalisées dans le même matériau.

11. Dispositif de mesure selon l'une quelconque des revendications 4 à 6 précédentes, **caractérisé en ce que** le trou d'interconnexion (12) et/ou l'élément plan (11) sont/est réalisé(s) en métal.

12. Dispositif de mesure selon la revendication 3 précédente, **caractérisé en ce que** le dispositif de mesure (1) comprend une unité d'énergie (13), une unité de mémoire et/ou interface (14), en particulier une interface NFC, qui sont disposées dans et/ou sur l'unité de support (9) et/ou l'unité de mesure (3).

13. Dispositif de mesure selon l'une quelconque des revendications précédentes, **caractérisé en ce que** la couche de protection (6) est réalisée en polyester et/ou en polyéthylène.
